# EUROPEAN PATENT APPLICATION

(11) **EP 2 135 954 A1**
(43) Date of publication of application: **23.12.2009**
(21) Application number: 08158500.2
(22) Date of filing: 18.06.2008
(51) Int. Cl.: C12P 7/62

(54) **Process for selecting polyhydroxyalkanoate (PHA) producing micro-organisms**

(71) Applicant: DSM IP Assets B.V., 6411 TE Heerlen (NL); Delft University of Technology, 2629 HS Delft (NL)
(72) Inventor: Loosdrecht Van, Marinus Cornelis Maria, 2628 BC Delft (NL); Kleerebezem, Robbert, 2497 XX Delft (NL); Muyzer, Gerard, 2151 LS Nieuw Vennep (NL); Jiang, Yang, 2611 HW Delft (NL); Johnson, Katja, 2635 JG Den Hoorn (NL)
(74) Representative: van der Ploeg, Antonius Franciscus M.J.

(57) **Abstract**

The invention relates to a process for selecting a polyhydroxyalkanoate (PHA) producing micro-organism from a natural source comprising a variety of micro-organisms, comprising steps of preparing a fermentation broth comprising the natural source and nutrients in water; creating and maintaining aerobic conditions in the fermentation broth; applying a feast-famine cycle comprising a feast phase and a famine phase, wherein the feast-famine cycle is started with feeding a carbon source to the fermentation broth, nutrients are fed in such amounts to the fermentation broth that the carbon source becomes limiting for the growth of the micro-organisms during the famine phase; the feast-famine cycle has a cycle time of at least 8 hours, and the feeding of the carbon source is performed during a small initial fraction of the feast famine cycle time; withdrawing a part of the micro-organisms during and/or at the end of the feast-famine cycle (referred to as solids withdrawal step); and repeating the feast-famine-cycle and the solids withdrawal step, such that an average solids retention time (SRT) of less than 4 days results.

The invention also relates to a mixed culture or isolated microorganism which has an activity of producing PHA, obtainable by the said selection process, use thereof in a process for producing a PHA, and the resulting PHA containing microorganism.

## Description

The invention relates to a process for selecting polyhydroxyalkanoate (PHA) producing micro-organisms from a natural source comprising a variety of micro-organisms. The invention also relates to a process for the production of polyhydroxyalkanoates.

Polyhydroxyalkanoates (PHA) have been recognized as good candidates for production of biodegradable plastics. Furthermore, PHA can be produced from renewable carbon sources, including wastewater streams, allowing for a sustainable and closed cycle process for the production and use of such polymers. PHAs are polyesters that are synthesized and stored within the cell by various organisms. Industrial processes based on pure cultures have initially been developed, but more recently the interest in the use of mixed cultures for the production of PHA has increased. Selection processes are applied to enhance the maximum obtainable PHA content with the mixed cultures. Though the use of mixed cultures may have economic advantages, the maximum obtainable PHA content for mixed cultures is still behind that of pure cultures.

In a review article by J.M.A. Dias et al, with the title of "Recent advances in Polyhydroxyalkanote Production by Mixed Aerobic Cultures: From Substrate to the Final Product" in Macromol. Biosci. 2006, 6, 885-906, a process comprising a pulse substrate feeding strategy is described. Such a process is also known as "aerobic dynamic feeding" process or "feast and famine" process. Herein an activated sludge is submitted to consecutive periods of external substrate availability (feast) and unavailability (famine). With such a process an intracellular PHA content of 65% can be reached. A similar process is described in patent application WO00/5218 The selection process of WO00/52189 is an aerobic process which comprises a first phase in which substrate is added, alternated with one in which substrate is withheld. The maximum PHA content obtained with that process is around 52 wt.%.

For a PHA production process with pure cultures to be economic, a high PHA content is essential. In the PHA production process with mixed cultures, the selection process to achieve a mixture enriched with micro-organisms with which a high PHA content can be obtained, is a costly process adding extra costs to the PHA production.

In view of the above there is a need for a process for selecting polyhydroxyalkanoate (PHA) producing micro-organisms from a natural source comprising a variety of micro-organisms that allow for a higher PHA production.

The aim of the invention is to provide such a selection process, and to provide a mixed culture comprising polyhydroxyalkanoate (PHA) producing micro-organisms that have higher PHA accumulating capacity.

This aim has been achieved with the selection process according to the invention, comprising the steps of
- preparing a fermentation broth comprising the natural source and nutrients in water;
- creating and maintaining aerobic conditions in the fermentation broth;
- applying a feast-famine cycle comprising a feast phase and a famine phase, wherein
   o the feast-famine cycle is started with feeding a carbon source to the fermentation broth;
   ○ nutrients are fed in such amounts to the fermentation broth that the carbon source becomes limiting for the growth of the micro-organisms during the famine phase;
   o the feast-famine cycle has a cycle time of at least 8 hours; and
   o the feeding of the carbon source is performed during a small initial fraction of the feast famine cycle time;
- withdrawing a part of the micro-organisms during and/or at the end of the feast-famine cycle (referred to as solids withdrawal step); and
- repeating the feast-famine-cycle and the solids withdrawal step, such that an average solids retention time (SRT) of less than 4 days results.

It has been observed that the process according to the invention, comprising feeding the carbon source in a short time thereby creating a period during which an excessive amount of carbon source is present, and using an extended feast-famine cycle thereby prolonging the famine phase, in combination with a short average solids retention time (SRT), results in a selection of micro-organisms that has an increased PHA production and accumulation capacity.

The carbon source is likewise initially converted into PHA, and subsequently through conversion of the PHA, used for growth of the micro-organisms. The carbon source is also indicated as carbon substrate.

The term "nutrients" is herein understood to comprise any substance, except the carbon source, that allows or contributes the growth of the micro-organism. The carbon source can be used for growth of the micro-organisms, either directly or indirectly, for example via PHA, but is within the context of the present invention explicitely excluded from the term nutrients.

Likewise by shortening the feed period and prolonging the famine phase, the period during which the carbon substrate is present in only very limited concentration or eventually completely absent, stimulates the growth of micro-organisms capable of producing PHA at high speed and in high concentration.

The feast-famine cycles in the process according to the invention may be prolonged over a very long time, although this will be limited by practical conditions. Preferably, the feast-famine cycles have a cycle time in the range of 8-24 hours, more preferably 10-15 hours. A longer feast-famine cycle time has the advantage that the selection process results in micro-organisms that have an even further increased PHA production and accumulation capacity, whereas a shorter feast-famine cycle time allows for a higher production activity. In terms of substrate conversion and biomass production

In the selection process, the long feast famine cycles as according to the invention may likewise been preceded by feast famine cycles with shorter cycle times. It has been observed by the inventors that a selection process with feast famine cycles of about 4 hours, which cycle sequence was maintained prolonged for a long time, resulted in micro-organisms capable of producing PHA in an maximum amount of about 60-72 wt.%(at 20°C). When the cycle time of the feast famine cycles was extended to 12 hours, and the temperature was increased to 30°C, within a limited number of cycles a new steady state was achieved resulting in micro-organisms capable of producing PHA in an amount of about 78-92 wt.%. Without the temperature being was increased, a similar increase was observed, while taking a larger number of cycles to reach the new steady state.

In the process according to the invention the carbon source is preferably fed with a feeding rate that is faster than the uptake rate, being the rate by which the carbon source is taken up by micro-organism and converted into PHA and/or used to maintain and/or grow the micro-organisms. This can be accomplished by further shortening the period during which the carbon substrate is fed.

Preferably, the feeding of the carbon source is performed in less than 20%, more preferably less than 10%, or even less than 5%, of the feast famine cycle time. Suitably, the carbon source is fed within a time span in the range of, for example, 5 to 30 minutes.

The nutrients are fed to the fermentation broth in such an amount that the carbon source becomes limiting for the growth of the micro-organisms during the famine phase. The nutrients can be fed together with the carbon source, but may also be fed separately or alternatively continuously during the feast-famine cycle, provided that their total content and individual concentrations of nutrient elements in the fermentation broth are always sufficiently high to prevent growth of the micro-organisms to be limited as a result of insufficient nutrients. Relevant nutrient elements include nitrogen (N), phosphorous (P), sulphur (S), whereas also potassium (K), and magnesium (Mg), might also be included. Furthermore, presence of trace elements like copper (Cu), iron (Fe), molybdenum (Mo), zinc (Zn), cobalt (Co), manganese (Mn), Calcium (Ca) may favour the growth of the microorganisms.

Suitable sources for N, P and S include ammonium chloride, ammonium sulphate and ammonium phosphate. Alternatively potassium and magnesium salts or other salts of sulphate, phosphate and nitrate might be added. The nutrients may be comprised in a waste stream that is used for the carbon source. The amount of nutrients needed for non-growth limiting conditions can be determined by the person skilled in the art by standard tests and routine experiment.

The concentration of micro-organisms in the fermentation broth can be varied over a wide range. The concentration of micro-organisms in the fermentation broth at the start of each feast-famine cycle may well be in the range of 0.1-100g/l, or even higher or lower. The initial concentration can be even much lower and only an inoculation step with some waste material or material from an environmental sample can be sufficient. The concentration of micro-organisms in the fermentation broth at the start of each feast-famine cycle preferably is in the range 0.5-50, 1-30, 2-20, or even better 5-10 g/l. A higher minimum concentration is more favourable for economical production, while a lower maximum concentration may be preferred for better process control.

During or at the end of each feast-famine cycle in the selection process an amount of solids is withdrawn from the reactor, by which a part of the micro-organisms, being the main component comprised by the solids, is withdrawn. These solids, primarily consisting of micro-organisms, optionally contain other components, such as PHA, substrate, nutrients and/or other solid components. At the end of the feast-famine cycle, the PHA content will be low, if any, but a small residual amount may still be present. The mass % of solids withdrawn is considered representative for the part of the micro-organism withdrawn. The solids withdrawal may be accomplished by withdrawal part of the fermentation broth, either homogenized or partly separated or concentrated, for example by prior sedimentation of the solids. The solids withdrawal is compensated by the surplus in carbon source and other nutrients fed in each subsequent feast famine cycle. Total amount of carbon source and other nutrients fed is always larger than amount of solids withdrawn since part of carbon source is converted into CO2 and used for energy supply.

The larger the amount of fermentation broth being removed together with the solids, the more water and other components have to be replenished together with or next to the feeding of the carbon source and the nutrients to compensate for the total volume of the fermentation broth. This all belongs to the standard skills of the persons acquainted with fermentation processes.

The amount of solids withdrawn during and/or at the end of each feast-famine cycle may vary over a wide range, provided this withdrawal complies with the short SRT of less than 4 days. Suitably, amount of solids withdrawn is in the range of 11-80 mass %, preferably 20-70 mass %, more preferably 30-65 mass %, and even 45-55 mass %, relative to the total amount of solids present at the end of the feast-famine cycle and withdrawn during the feast-famine cycle.

Suitably the SRT is in the range of 12 hours - 3 days, preferably 18 hours - 2 days, more preferably from 20 to 36 hours.

The larger the amount of solids being withdrawn in each feast-famine cycle, the lower is the number of feast-famine cycles (FFC) per average solid retention time (SRT), expressed as FFC/SRT ratio. Suitably, the FFC/SRT ratio is in the range of 1.2 - 6, though a ratio higher than 6 as well as a ratio lower than 1.2 may be used. Preferably, the ratio is in the range of 1.5 - 4, more preferably 1.8 - 2.5. A ratio of 2, corresponding with an average solids withdrawal per feast famine cycle of 50 wt.% has been observed to allow for a fast and efficient selection of the improved/selected organism with a high growth rate and high productivity of PHA.

The conditions, such as temperature and pH, under which the process according to the invention can be performed can vary over wide ranges. Suitably, the temperature at which the fermentation broth is kept during the feast-famine cycles is in the range of 10°C - 45°C, preferably 20- 40 °C, more preferably 25-35°C. Also the pH during the feast-famine cycles is kept suitably in the range of 3 - 11, although preferably is in the range of 5 - 9, more preferably 6 - 8. A combination of a pH around 7 and a temperature around 30 °C during the feast-famine cycles gave very good results.

It is realized that the medium might have a certain buffer capacity which will not be enough to keep the pH stable, so some variations may occur, especially if salts of the acids are used as feed rather than the acids - i.e. it depends on the medium composition whether additional pH control is needed or not. The actual pH control may be performed for example by using buffers, such as ammonium sulphate and ammonium phosphate or by addition of acid (e.g. HCl) and if needed a base (e.g. NaOH) addition. Addition of the carbon source as an acid rather than a salt has the advantage that less pH regulator has to be added.

The process according to the invention results in micro-organisms so effectively that already after 2-4 feast famine cycles, a clear feast-famine pattern in the reactor can be observed. The number of cycles needed for the micro-organisms to be sufficiently enriched to be collected in the withdrawal step for use of polyhydroxyalkanoate production with high PHA accumulation, depends on the starting inocculum. Starting from an from a mixed culture, that had been enriched before by means of other processes will take less cycles, e.g. in the range of 2-4 cycles, than starting from an activated sludge, which will take at least 10 cycles

Further improvement is obtained after increasing the number of feast-famine cycles, however it takes only a limited number of cycles to reach a close-to-steady-state situation. The close-to-steady-state situation may be reached already after 10 to 30 cycles, and collection may be started already by then. The process is very stable and may be continued for many cycles for a very long time. It was observed that the PHA production capacity of the culture improved when the feast famine process was continued over a long time. Without the intention of being limitative, the process may well be continued for 5,000 - 10,000 cycles, or even more.

The term close-to-steady-state situation is herein used, because the differences between the subsequent cycles are so minimal that they are hardly visible if not invisible at all. However, at the long run there still is a gradual but continuous improvement in PHA production capacity of the selection improved micro-organism observed. Therefore, it is advantageous to proceed the process for long time. Meanwhile the micro-organisms withdrawn from the fermentation broth can be used for PHA production, thus making optimal use of the carbon sources used in the selection process and the PHA production process. The result is that the carbon efficiency of the combined selection process and PHA production process becomes very high.

For obtaining a steady state situation, it simply requires to feed in each feast famine cycle about the same amount of carbon substrate and to remove in each cycle about the same percentage of the reactor liquid and solids. The system is self-regulating. In such a self-regulating system under steady state, the amount of biomass formed is equal to the amount of biomass removed in each cycle. For the process according to the invention, however, the amounts of carbon substrate added and the amounts of the reactor liquid and solids removed in each cycle do not need to be exactly the same every cycle. The person skilled in the art of fermentation processes will be able based on routine experiments to find condictions to run the process as a stable process.

The carbon substrate that is fed in the process according to the invention may be any carbon substrate that is suitable for and can be converted into a PHA. The carbon source may comprise, for example, an alkanoic acid, such as a fatty acid, an alkanol, a hydroxyl functional alkanoic acid, or a mixture thereof, or a salt of the acids. An example of a suitable hydroxyl functional alkanoic acid is lactic acid. Preferably, the carbon source is limited in molecular weight, more preferably the carbon source comprises at least one of volatile fatty acids with 2-9 C-atoms. Also more preferably the fatty acid, the alkanol, respectively the hydroxyl functional alkanoic acid comprises at most 9 carbon atoms, more preferably at most 6 carbon atoms. Suitably the number of carbon atoms is one of 2, 3, 4 or 5. Examples of suitable alkanoic acids, or low volatile fatty acids, with these numbers of carbon atoms, that can be used in the process according to the invention are acetic acid (C2), propionic acid (C3), butyric acid (C4) and valeric acid (C5). These low volatile fatty acids may be used as such or as a salt, being an acetate, propionate, butyrate, valerate or any mixture thereof, or as mixture with other nutrients. Suitably the salt is a sodium or potassium salt. Preferably, the carbon source comprises acetic acid or sodium acetate.

The selection process according to the invention can be carried out in a reactor. Suitably such a reactor is a fermentor, or any other reactor, that is suited for carrying out a fermentation process. The reactor may be equipped with stirring equipment, inlets and outlets for solid, liquid and gaseous components. Otherwise the reactor may even be an open reactor, since the process may be carried out under non-sterile conditions. Aerobic conditions may be created and further maintained, for example, by supplying an oxygen comprising gas, by stirring of the fermentation broth under atmospheric conditions or elevated pressure, by adding nitrate, or a combination thereof. The elevated pressure suitably is an overpressure of less than 50 kPa relative to an ambient pressure of 100 kPa.

The selection process according to the invention cannot only be used for selecting PHA producing micro-organisms from a natural source comprising a variety of micro-organisms. The selection process can also be used to optimize the PHA production capacity of sources comprising a variety of micro-organisms, other than natural sources. The selection process can also be used to optimize the metabolism for PHA production in pure cultures. In particular this will apply for pure cultures of natural producers and GMO's of natural producers, since these have the genes for PHA degradation und therefore will also grow in the famine phase. Thus in the process with the feast-famine cycles according to the invention, the natural source may also be replaced by other than natural sources comprising a variety of micro-organisms or by PHA producing pure cultures.

The process with the feast-famine cycles according to the invention may be modified into a growth process for increasing the total amount of biomass of PHA producing micro-organisms, rather than for selection of PHA producing micro-organisms from the biomass. This modification only requires reduction of the number and/or size of the the solids withdrawal step and repeating a couple of times the feast-famine cycles.
The invention also relates to a process for producing a PHA, and to a mixed culture or isolated micro-organism which has an activity of producing PHA, that can be used in said production process.

In particular, the mixed culture or isolated micro-organism having activity of producing PHA is obtainable by the selection process according to the invention.
In one embodiment of the invention, the said mixed culture or isolated micro-organism is obtainable by the selection process according to the invention, wherein the number of the feast-famine cycles is at least 5. Such a mixed culture or isolated micro-organism has an increased activity and capacity for producing PHA.

In another embodiment of the invention the said mixed culture or isolated micro-organism has an activity of producing PHA of 0.5 to 5 gram PHA per gram micro-organism (on dry matter) in the first hour after feeding of the carbon substrate at 30°C. Suitably, the PHA producing activity is in the range of 0.75 to 2 gram PHA per gram micro-organism (on dry matter) in the first hour after feeding of the carbon substrate at 30°C

In a further embodiment, the said mixed culture or isolated micro-organism has a PHA accumulation capacity of at least 73 %wt, relative to total weight of PHA and micro-organism. Preferably, the mixed culture or isolated micro-organism has a PHA accumulation capacity in the range of 75-95 wt.%, and suitably in the range of 80-92 wt.%.

The mixed culture or isolated micro-organism in the above embodiments preferably comprises 50-100 wt.%, of a micro-organism belonging to the class of *Gammaproteobacteria* which has an activity of producing PHA. The obtained mixed culture may also be used to isolate a PHA producing strain belonging to the class of *Gammaproteobacteria.*

Preferably the said *Gammaproteobacteria* is a strain deposited under nr CBS 122990 Bacterial strat TUD-YJ37 at the Centraalbureau voor Schimmelcultures (CBS), Uppsalalaan 8, P.O. Box 85167, 3508 AD UTRECHT, The Netherlands, under the Budapest Treaty on the International Recognition of the Deposit of Micro-organisms for the Purposes of Patent Procedure Statement in the Case of an Original Deposit Pursuant to Rule 6.1.

Using the micro-organism or enriched mixed culture comprising the said *Gammaproteobacteria* with said strain in a PHA production process it is possible to achieve a maximum PHA content within 10 hours. With a pure culture of GMO *E.coli* the similar maximum level of PHA accumulation can be achieved but this takes 40 hours.

The enriched mixed microculture obtained with the selection process preferably comprises the the said *Gammaproteobacteria* with said strain deposited under nr CBS 122990 Bacterial strat TUD-YJ37 in an amount of at least 50 wt.%, more preferably at least 70 wt.%, or 80 wt.% or even better at least 90 wt.%, relative to the total weight of the mixed microculture. Most preferably, the *Gammaproteobacteria* with said strain deposited under nr CBS 122990 Bacterial strat TUD-YJ37 is a pure microculture. Such pure microculture might be isolated from a mixed microculture obtained with the selection process according to the invention. Such isolated pure microculture might be cultivated and grown for use in PHA production processes, as well as in other fermentation processes.

The invention also relates to a process for producing a PHA, comprising feeding of a carbon substrate to a fermentation broth comprising a micro-organism, followed by conversion of the carbon source by the micro-organism into the PHA. In the inventive process, the micro-organism comprises a mixed culture or isolated micro-organism according to the present invention described above.

This process may be used, for example, as a fermentation process for the production of a mixture of PHA's or specific PHAs, or alternatively, for wastewater treatment wherein wastewater comprising carbon substrates is used. For the carbon substrate, also oil containing natural sources may be used, such as palm oil, palm oil waste material, olive oil. Preferably the carbon substrate in this process comprises a fatty acid, such as the ones described above for the selection process. More preferably, the carbon substrate is acetic acid or a salt thereof and the process is used for producing polyhydroxybutyrate (PHB).

The said process may be performed under a wide variety of different circumstances. As an indication, but without being limited thereto, the process may be carried out at a temperature in a range of 10°C - 45°C, preferably 20- 40 °C, more preferably 25-35°C, and/or at a pH in the range of 3 - 11, preferably 5 - 9, more preferably 6-8.

The PHA production process is preferably performed under growth limiting conditions, but not necessarily so. The growth may be limited by one or more nutrients, for example, by N, P and/or S. In a preferred embodiment of the invention, the nutrients comprise nitrogen in a limited amount, if any, such that the molar ratio of N/C is in the range of 0- 1/8, preferably 0-1/40, and still more preferably 0-1/200, wherein N is the molar amount of nitrogen atoms in the nutrients and C is the molar amount of carbon atoms in the carbon source. By creating growth limiting conditions, in particular by reducing the amount of ammonia, the PHA content is further increased and the amount thereof declines less with longer operating times.

The PHA production process according to the invention also allows production of PHA in high quantities and in short time. Preferably, the process comprises a step wherein solid material comprising the micro-organism and the PHA are isolated from the fermentation broth within a time span of 1.5 - 15 hours, preferably 2 - 10 hours, 4-8 hours, after feeding of the carbon source. This short time needed for obtaining optimum PHA production can ideally be combined with the short SRT times provided by the inventive selection process and allows for optimal use of fermentor capacity for both the selection process and the PHA production.

The mixed culture or isolated micro-organism that is used in the PHA production process according to the invention, preferably comprises a micro-organism belonging to the class of *Gammaproteobacteria* obtained by the inventive selection process described above. More preferably the said *Gammaproteobacteria* is a strain deposited under nr CBS 122990 Bacterial strat TUD-YJ37 at the CBS in Utrecht

The PHA production process according to the invention can be carried out in any equipment and any set-up suitable for fermentation processes, and can be carried out, for example, as a fed-batch or batch process.

The invention also relates to a PHA containing micro-organism, obtainable from the PHA production process according to the invention, having a PHA content of at least 73 wt.%, relative to total weight of PHA and micro-organism. Suitably, the PHA-content in the PHA containing micro-organism is in the range of 75-95 wt.%, and eventually in the range of 78-92 wt.%, relative to total weight of PHA and micro-organism. The micro-organism in the said PHA containing micro-organism preferably comprises the *Gammaproteobacteria* is a strain deposited under nr CBS 122990 Bacterial strat TUD-YJ37 at the CBS in Utrecht. More preferably, the relative amount of said *Gammaproteobacteria* is at least 50 wt.%, relative to the total weight pf the micro-organism. Most preferably, the micro-organism consist of the pure or isolated *Gammaproteobacteria.*
Fig 1. Graphical representation of analytical data for a single feast-famine cycle.
Fig 2. Graphical representation of PHA accumulation during a PHA production cycle.

The invention is further illustrated with the following experiments and comparative examples

### Experimental

### Selection process

### Comparative Experiment A

For the selection process a 21 sequencing batch reactor (SBR) with a cycle length of 4h, a SRT of 4d and a hydraulic retention time (HRT) of 8h was used. The starting medium was inoculated with activated sludge from a waste water treatment facility.
The medium had the following composition: Carbon source: 0.82g/l NaAc·3H₂O; Nutrients: 0.107g/l NH₄Cl, 0.226g/l KH₂PO₄, 0.091g/l MgSO₄·7H₂O, 0.036g/l KCI, 1ml/l trace elements solution according to Vishniac & Santer (W. VISHNIAC and M. SANTER, The Thiobacilli, Bacteriol.Rev. 21 (3):195-213, 1957) and 0.3ml/l allylthiourea (to prevent nitrification). Each feast famine cycle consisted of a start phase (running from 0-10min), feeding phase (10-13 min), reaction phase (13-216min), solids withdrawal phase (216-218min), settling phase (218-233min) and a effluent withdrawal phase (233-240min).

The reactor temperature was maintained at 20°C. The pH was controlled at 7 ± 0.1 with the addition of acid (1 M HCl) and base (1 M NaOH). The reactor was fully aerated (airflow 1.8 l/min) apart from the settling phase and the solids and effluent withdrawal phases. The reactor was stirred with stirrer speeds of 625 rpm for 30 minutes starting from the beginning of the feeding phase and 310 rpm in the start phase, remainder of the reaction phase and during solids withdrawal. The process was continued for 0.5 year. Subsequently the nitrogen/carbon ratio of the supplied medium was changed from 6 mol/mol to 24, 11 and 15 over a time frame of about 1 year.

The culture obtained from the last withdrawal steps for each of the different nitrogen/carbon ratio experiments was used for PHA accumulation experiments. In these experiments a polyhydroxybutyrate (PHB) content of 61-71 wt.% was obtained. No correlation between maximum amount of PHB and nitrogen/carbon ratio was found.

### Comparative Experiment B

Comparative experiment A was modified by using samples from Comparative Experiment A as an inoculum, and applying a SRT of 1d. The medium had the following composition: Carbon source: 2.45g/l NaAc·3H₂O; Nutrients: 0.241g/l NH₄Cl, 0.226g/l KH₂PO₄, 0.091g/l MgSO₄·7H₂O, 0.036g/l KCl, 1ml/l trace elements solution according to Vishniac & Santer (W. VISHNIAC and M. SANTER, The Thiobacilli, Bacteriol.Rev. 21 (3):195-213, 1957) and 0.3ml/l allylthiourea (to prevent nitrification). No improvement in PHB content was observed.

### Example I

For the selection process of Example I, biomass selected from the last withdrawal steps of Comparative Experiment B was used as inoculum. The SBR cycle length was increased to 12h and settling and effluent withdrawal phases were omitted (SRT = HRT = 1d). Each feast famine cycle consisted of a start phase (0-7min), feeding phase (7-17 min), reaction phase (17-700min) and solids withdrawal phase (700-720min). The reactor temperature was increased to 30°C.

The medium had the following composition: Carbon source: 3.67g/l NaAc-3H₂O;
Nutrients: 0.361g/l NH₄Cl, 0.339g/l KH₂PO₄, 0.137g/l MgSO₄·7H₂O, 0.054g/l KCI, 1.5ml/l trace elements solution according to Vishniac & Santer (W. VISHNIAC and M. SANTER, The Thiobacilli, Bacteriol.Rev. 21 (3):195-213, 1957) and 0.3ml/l allylthiourea (to prevent nitrification).

The pH was controlled at 7 ± 0.1 with the addition of acid (1 M HCl) and base (1 M NaOH). The reactor was fully aerated apart from the solids withdrawal phase. A gas recirculation system was applied with a flow of fresh air supplied to the reactor of 0.2 l/min and a recycle stream of 1.1-1.2 l/min, resulting in a gas flow of 1.3-1.4 l/min through the reactor. The reactor was stirred with stirrer speeds of 580 rpm (for 5 minutes) and 1000 rpm (for 5 minutes) during the feeding phase and with 750 rpm for the remainder of the cycle. The process was continued for about 1.5 years.

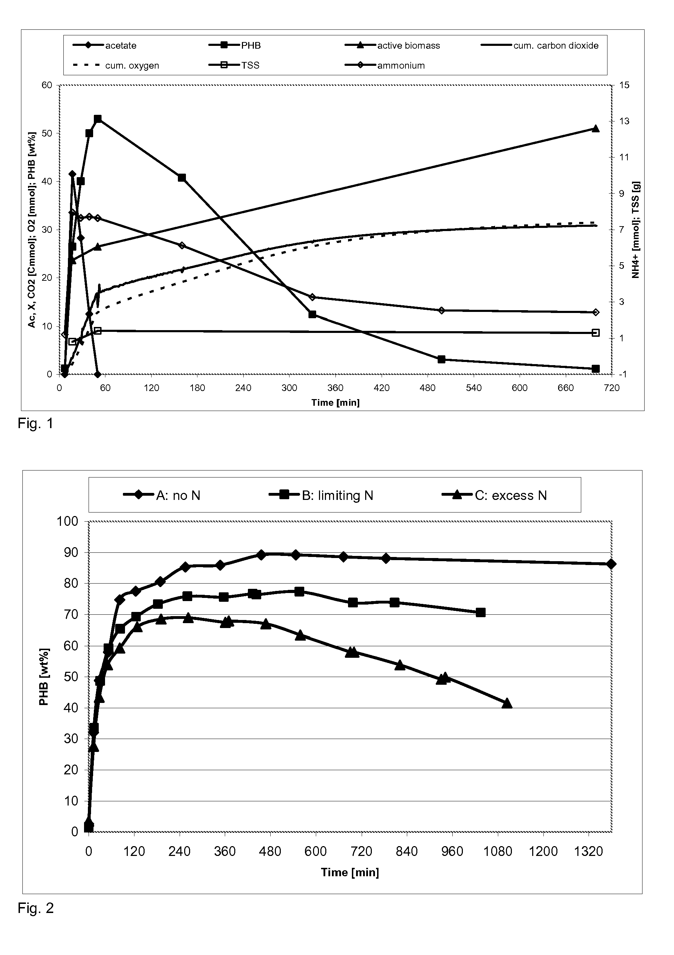

A graphical representation of analytical data for a feast famine cycle is displayed in figure 1.

Figure 1 shows a graphical representation of analytical data for a single feast-famine cycle, from which the feed of the acetate and subsequent disappearance thereof is visible, as well as the initial increase in PHB and subsequent gradual decrease thereof, and the gradual increase in biomass content.

The culture obtained from the last withdrawal steps was used for the PHA accumulation experiments in Example II.

### Microbial diversity analysis

During the experiment of Example I, the microbial diversity of the mixed culture obtained with the selection process was studied using Denaturing Gradient Gel Electrophoresis (DGGE). Herein, one dominating micro-organism was found. Based on its 16S rRNA, this micro-organism is a member of the *Gammaproteobacteria,* but it is quite dissimilar from its closest known relatives. The dominance of this organism found with DGGE was confirmed using Fluorescence In Situ Hybridisation (FISH) with a specific probe designed for this organism. The mixed culture was stained with fluorescein-labelled 16S rRNA probe for Eubacteria (EUB338, red) and Cy5-labelled 16S rRNA probe for the dominant community member (UCB823, green). The dominant organism was yellow, indicating that both probes hybridized.

### Isolation of the monoculture.

Form the mixed culture obtained in Example I, a monoculture of the dominant micro-organism was isolated. This monoculture, which was identified as a *Gammaproteobacteria* is deposited under nr CBS 122990 Bacterial strain TUD-YJ37 at the Centraalbureau voor Schimmelcultures (CBS), Uppsalalaan 8, P.O. Box 85167, 3508 AD UTRECHT, The Netherlands, under the Budapest Treaty on the International Recognition of the Deposit of Micro-organisms for the Purposes of Patent Procedure Statement in the Case of an Original Deposit Pursuant to Rule 6.1.

### PHA accumulation experiments

### Example II

For the PHA accumulation experiments described below. Herein, the culture obtained from the last withdrawal steps in Example I, was used for the PHA accumulation in a fed-batch reactor.

The same temperature, pH, stirrer speed and aeration as in example 1 were applied. The fed-batch reactor was in each case supplied with 1l of biomass and 1l of acetate-and ammonium-free medium composed of 0.339g/l KH₂PO₄, 0.137g/l MgSO₄·7H₂O, 0.054g/l KCl, 1.5ml/l trace elements solution according to Vishniac & Santer (W. VISHNIAC and M. SANTER, The Thiobacilli, Bacteriol.Rev. 21 (3):195-213, 1957) and 0.3ml/l allylthiourea (to prevent nitrification). The experiments were started by supplying a pulse of 120 Cmmol (60 mmol) NaAc to the fed batch reactor. Subsequent feeding was implemented using the pH control. The feed (i.e. acid for pH control) was in experiment A (no nitrogen source) 1.5M acetic acid. In experiment B (nitrogen limited, N:C = 1 mol:40mol) the feed was composed of 1.5M acetic acid including 4.02g/l NH₄Cl, 3.76g/l KH₂PO₄, 1.52g/l MgSO₄·7H₂O, 0.60g/l KCl, 16.66ml/l trace elements solution according to Vishniac & Santer (W. VISHNIAC and M. SANTER, The Thiobacilli, Bacteriol.Rev. 21 (3):195-213, 1957) and 3g NaOH. In experiment C (nitrogen excess, N:C = 1mol:8mol) the feed was composed of 1.5M acetic acid including 20.06g/l NH₄Cl, 18.8g/l KH₂PO₄, 7.6g/l MgSO₄·7H₂O, 3.0g/l KCl, 83.3ml/l trace elements solution according to Vishniac & Santer (W. VISHNIAC and M. SANTER, The Thiobacilli, Bacteriol.Rev. 21 (3):195-213, 1957) and 6g NaOH.

In each of the 3 experiments, at different time intervals samples were taken and the PHB content of solids was measured and determined as a percentage of the total solids, i.e. including both the micro-organism and the PHB. The results are included in the diagram below (Fig 2).

Figure 2 shows a graphical representation of PHA accumulation as a function of time during the PHA production cycles of experiments A,B and C of Example II. As is shown, all 3 experiments reach a PHB content above 65% already within 2 hours. With experiment (A), i.e. in the experiment without any N, a PHB content of 89.2 wt% was reached after 7.6 hours. In the other two experiments the maximum content is lower, and with increasing N content, the PHB content decreases upon longer processing times. Apparently by reducing the amount of ammonia the PHB content is further increased and the amount thereof declines less with longer operating times. Apparently, even under conditions with rather large amounts of nutrients present, a high PHB content can be reached. This content is obtained within a limited number of hours. To be able to obtain the maximum amount of PHB under all conditions, the process might be stopped after a relatively short time.

## Claims

1. A process for selecting a polyhydroxyalkanoate (PHA) producing micro-organism from a source comprising a variety of micro-organisms, comprising steps of
- preparing a fermentation broth comprising the source comprising the variety of micro-organisms and nutrients in water;
- creating and maintaining aerobic conditions in the fermentation broth;
- applying a feast-famine cycle comprising a feast phase and a famine phase, wherein
o the feast-famine cycle is started with feeding a carbon source to the fermentation broth,
○ nutrients are fed in such amounts to the fermentation broth that the carbon source becomes limiting for the growth of the micro-organisms during the famine phase;
o the feast-famine cycle has a cycle time of at least 8 hours, and
o the feeding of the carbon source is performed during a small initial fraction of the feast famine cycle time;
- withdrawing a part of the micro-organisms during and/or at the end of the feast-famine cycle (referred to as solids withdrawal step); and
- repeating the feast-famine-cycle and the solids withdrawal step, such that an average solids retention time (SRT) of less than 4 days results.

2. The process according to claim 1, wherein the feast-famine cycles have a cycle time in the range of 8-24 hours, preferably 10-15 hours.

3. The process according to claim 1 or 2, wherein the SRT is in the range of 12 hours - 3 days, preferably 18 hours - 2 days.

4. The process according to any of claims 1-3, wherein during the feast-famine cycle the fermentation broth is kept at a temperature in the range of 10°C - 45°C, and/or at a pH in the range of 3 - 11.

5. The process according to any of claims 1-4, wherein the carbon source comprises a fatty acid, a fatty alcohol or a fatty acid alcohol, or a mixture thereof.

6. A mixed culture or isolated microorganism which has an activity of producing PHA, obtainable by the selection process according to any of claims 1-5, wherein the number of the feast-famine cycles is at least 5.

7. A mixed culture or isolated microorganism obtainable by the selection process according to any of claims 1-6, which has an activity of producing PHA of 0.5 to 5 gram PHA per gram microorganism (on dry matter) in the first hour after feeding of a carbon substrate at 30°C.

8. A mixed culture or isolated microorganism obtainable by the selection process according to any of claims 1-5, having a PHA accumulation capacity of at least 73 %wt, preferably in the range of 75-95 wt.%, relative to total weight of PHA and microorganism.

9. A mixed culture or isolated microorganism according to any of claims 6-8, comprising a microorganism belonging to the class of *Gammaproteobacteria* which has an activity of producing PHA.

10. A mixed culture or isolated microorganism according to claim 9, wherein the *Gammaproteobacteria* is a strain deposited under nr CBS 122990 Bacterial strain TUD-YJ37 at the Centraalbureau voor Schimmelcultures (CBS), Uppsalalaan 8, P.O. Box 85167, 3508 AD UTRECHT, The Netherlands, under the Budapest Treaty on the International Recognition of the Deposit of Micro-organisms for the Purposes of Patent Procedure Statement in the Case of an Original Deposit Pursuant to Rule 6.1.

11. A process for producing a polyhydroxyalkanoate (PHA), comprising feeding of a carbon substrate and optionally feeding nutrients to a fermentation broth comprising micro-organisms, followed by allowing conversion of the carbon source by the micro-organisms into the PHA, wherein the micro-organisms comprise a mixed culture or isolated microorganism according to any of claims 6-10.

12. The process according to claim 11, wherein the nutrients comprise nitrogen in a limited amount, if any, such that the molar ratio of N/C is in the range of 0- 1/8, wherein N is the molar amount of nitrogen atoms in the nutrients and C is the molar amount of carbon atoms in the carbon source.

13. The process according to claim 11 or 12, comprising a step wherein solid material comprising the micro-organism and the PHA are isolated from the fermentation broth within a time span of 1.5 - 15 hours, after feeding of the carbon source commenced.

14. The process according to any of claims 11-13, wherein the polyhydroxyalkanoate (PHA) is polyhydroxybutyrate (PHB) or wherein the process is used for wastewater treatment.

15. A polyhydroxyalkanoate (PHA) containing microorganism, comprising a mixed culture or isolated microorganism according to any of claims 6-10 and/or obtainable from the process according to any of claims 11-14, having a PHA content of at least 73 wt.%, relative to total weight of PHA and microorganism.
